Europäisches Patentamt

European Patent Office

Office européen des brevets

(19)

(11) Publication number : **0 578 376 A1**

(12) # EUROPEAN PATENT APPLICATION

(21) Application number : **93304462.0**

(22) Date of filing : **09.06.93**

(51) Int. Cl.⁵ : **A61B 17/32, A61M 1/00**

(30) Priority : **18.06.92 GB 9212922**

(43) Date of publication of application :
**12.01.94 Bulletin 94/02**

(84) Designated Contracting States :
**DE FR IT**

(71) Applicant : **SPEMBLY MEDICAL LIMITED**
**Newbury Road**
**Andover, Hampshire SP10 4DR (GB)**

(72) Inventor : **Varney, Kelvin John**
**1 Fairpiece Cottage Wherwell, Andover**
**Hampshire SP11 7JD (GB)**

(74) Representative : **Robinson, Nigel Alexander**
**Julian et al**
**D. Young & Co., 21 New Fetter Lane**
**London EC4A 1DA (GB)**

(54) **Ultrasonic surgical aspirator.**

(57)    An ultrasonic surgical aspirator comprises a vacuum source (13), and a handpiece (17), the handpiece (17) comprising an aspiration tip (22) with an aspiration aperture (24) for communicating with the vacuum source (13), an ultrasonic transducer (23) for generating ultrasonic vibrations, means (18) for transmitting the ultrasonic vibrations to the aspiration tip (22), and control means (40,41) for varying the vacuum pressure at the aspiration aperture (24).

FIG.2a

FIG.2b

FIG. 2a | FIG. 2b

FIG.5

This invention relates to ultrasonic surgical aspirators.

In some minimally-invasive surgical operations ultrasonic surgical aspirators are used for selectively dissecting, emulsifying and removing unwanted biological material (e.g. tumours) from a patient's body tissue. Known ultrasonic surgical aspirators comprise an elongate handpiece having an ultrasonically vibrating tip. The handpiece includes at least two through passages for providing irrigation to the vibrating tip (e.g. by supplying an isotonic solution) and for aspirating (applying suction to) the vicinity of the vibrating tip. Aspiration performs two main functions: one is to help to maintain the tip in contact with the appropriate area of tissue to ensure effective emulsification of that area, and the other is to remove emulsified tissue, blood and surplus irrigant.

During some endoscopic surgical procedures (involving the visual examination of the interior of a body cavity), an enclosed volume of a patient's body is inflated to provide easier access for the surgeon's instruments. In particular, in laparoscopic surgical procedures (involving the visual examination of the interior of a patient's abdomen) a so-called insufflator is used to pump a gas (e.g. carbon dioxide) into a patient's abdomen at a positive pressure of up to about 12-14 millimetres of mercury (mmHg) to form a so-called pneumoperitoneum. Insufflators constantly monitor the patient's intra-abdominal gas pressure, stop further gas flow once a certain preset pressure is reached, indicate the flow rate of gas into the patient's abdomen, and record the total volume of gas delivered over a period of time.

The handpiece of an ultrasonic surgical aspirator, in common with other surgical instruments, is inserted into a patient's abdomen through an introducer cannula. This is an artificial tube inserted into the abdomen through a small incision at the start of a surgical operation. Once a cannula has been installed, surgical instruments can be inserted and withdrawn via the cannula with relative ease. Cannulae may be fitted with one-way valves which are opened by the insertion of a surgical instrument (to allow that instrument to enter the body) but which close automatically when an instrument is withdrawn to prevent the escape of the gas used to inflate the abdomen. However, despite these measures, gas may be lost from the patient's abdomen through leaks around the valves and seals of cannulae and during the exchange of instruments in a single cannula.

During the use of conventional ultrasonic surgical devices the aspiration vacuum level is normally preset at a vacuum pump control console, remote from the surgeon. Vacuum levels of up to 700 mmHg and free air flows of up to 25 l/min are achievable with the vacuum pumps generally fitted to such equipment, although the restriction imposed by the aspiration passage from the handpiece tip to the pump may reduce the free air flow to around 15 l/min. The working vacuum level is set at the start of a surgical operation and may be varied from time to time (though generally infrequently) during the operation by one of the operating theatre staff in order to achieve a level of vacuum appropriate to the type of tissue being removed and its location within the body.

A feature of conventional ultrasonic surgical devices is to provide controllable momentary tissue release. Under the control of, for example, a footswitch, the aspiration vacuum is shut off for a short period to release the tissue which may be held on the handpiece's tip and thus to allow the handpiece to be withdrawn from the operating site. This momentary release of vacuum is conventionally about one second so that the safe removal of the aspirator's tip is possible, without significantly affecting the continuous removal of tissue, blood and irrigant in the tip's vicinity.

Many older insufflators in current use have flow rates of only 3 litres per minute (l/min). Currently manufactured insufflators can generally provide flow rates of 6 l/min, or in some cases up to 8 to 10 l/min. Insufflators of these capacities are satisfactory for laparoscopic surgery in which ultrasonic surgical aspirators are not used, since the capacity of the insufflators can match the rate of gas extraction and loss. However, if an ultrasonic surgical aspirator is used for laparoscopic ultrasonic surgery, aspiration may be applied for extended periods of time which are sufficiently long that the pneumoperitoneum can be deflated by the aspirator. In other words, the aspirator removes gas from the abdomen faster than the insufflator can replace it. This problem can arise not only because a surgeon may require to aspirate an area for a length of time to perform a particular surgical procedure; the surgeon may also leave the ultrasonic aspirator inserted in the patient while he performs other surgical actions elsewhere on the patient to avoid having to remove and re-insert the ultrasonic aspirator.

This invention provides an ultrasonic surgical aspirator comprising a vacuum source and a handpiece, the handpiece comprising: an aspiration tip with an aspiration aperture for communicating with the vacuum source; an ultrasonic transducer for generating ultrasonic vibrations; means for transmitting the ultrasonic vibrations to the aspiration tip; and control means for varying the vacuum pressure at the aspiration aperture.

The invention recognises that applying unnecessary aspiration can lead to gas being removed from an operating site faster than it is replaced by an insufflator. This can result in the potentially hazardous deflation of the operating site. The invention addresses this problem by providing an ultrasonic surgical aspirator in which the handpiece has means for regulating the vacuum pressure used for aspiration from the vicinity of the aspirator tip. In this way control over the aspiration vacuum is given to the surgeon, who can therefore reduce the time during which unnecessary aspiration is applied.

Preferably the aspirator comprises an irrigant pump for supplying irrigant fluid to the handpiece and the handpiece comprises an irrigant outlet aperture for communicating with the irrigant pump. In particular, it is preferred that the aspirator comprises means for supplying a pulsed flow of irrigant fluid to the irrigant outlet aperture.

Although the control means may comprise, for example, an electrical control switch mounted on the handpiece, in a preferred embodiment the control means comprises means for restricting a path of fluid flow between the aspiration aperture and the vacuum source, so as to vary the vacuum pressure at the aspiration aperture. The path restriction may be achieved in a number of different ways, such as by means of a conventional gate valve, but it is preferred that the handpiece is connected to the vacuum source by a flexible aspiration hose, and that the control means comprises means for crimping the aspiration hose to restrict a path of fluid flow between the aspiration aperture and the vacuum source. Preferably the crimping means comprises a roller movable with respect to the aspiration hose between a crimping position and a non-crimping position. In order that an operator (e.g. a surgeon) can reduce the aspiration vacuum and then leave the surgical aspirator unattended, it is preferred that the handpiece comprises means for latching the roller in the crimping position.

In other preferred embodiments the control means comprises means for bleeding air to the vacuum source in order to vary the vacuum pressure at the aspiration aperture. Although this could be achieved by employing e.g. a hand-operated three-way valve mounted on the handpiece, it is preferred that the handpiece comprises a bleed aperture which, in use, can be partly or fully (i.e. at least partly) closed by covering with a finger or can be opened to bleed air to the vacuum source. Naturally, "full closure" of the bleed aperture may be limited by slight leakage around the operator's finger. This arrangement allows a controllable bleed of air to the vacuum source, without the need for any additional moving parts on the handpiece. It provides a degree of tactile feedback, to the operator's finger, of the changes in aspiration vacuum being made. Also, the "default" mode of operation (i.e. when the operator's finger is not covering the bleed aperture) is that air is bled to the vacuum source. This means that if the operator wishes to leave the handpiece unattended for a period, the aspiration vacuum is reduced by the air bleed during that period.

Many alternatives are possible for the shape of the bleed aperture. For example, a round aperture may be employed to give a simple on/off control over the vacuum pressure at the aspiration aperture. Alternatively a plurality of spaced bleed apertures may be employed so that the operator's finger can selectively cover one or more of the apertures to achieve a variable control over the vacuum at the aspiration aperture. A similar effect can be achieved in another preferred configuration in which an elongate aperture is used.

Although the bleed aperture may be formed as part of the handpiece itself, in a preferred embodiment the control means comprises a bleed tube attached to the handpiece, the bleed tube comprising: a first aperture for communicating with the vacuum source; a second aperture connected to the handpiece for communicating with the aspiration aperture in the handpiece; and a branching tube which, in use, overlies a gripping portion of the handpiece, the branching tube comprising a bleed aperture which, in use, can be partly or fully closed by covering with a finger or can be opened to bleed air to the vacuum source. In this way, control over the aspiration vacuum can be provided by means of a bleed tube attached to an otherwise conventional ultrasonic handpiece.

In a preferred embodiment the transmitting means is housed in an elongate prism-shaped shroud (i.e. a shroud having a constant cross-section and parallel sides, such as a cylindrically shaped shroud). This arrangement makes the handpiece particularly suitable for laparoscopic use, in which the shroud can be inserted through, and seal with, an introducer cannula. It should be noted that the transmitting means could also operate to increase the amplitude of the ultrasonic vibrations.

Although the handpiece may be used merely for ultrasonic aspiration, it is preferred to combine further surgical functions into a single instrument in order to reduce the number of cannulae which have to be inserted into a patient. In particular, in a preferred embodiment the handpiece is connectable to an electrocautery signal generator, the aspiration tip forming an electrocautery electrode when the handpiece is connected to the electrocautery signal generator.

Viewed from a second aspect this invention provides a handpiece for use in an ultrasonic surgical aspirator, the handpiece comprising: an aspiration tip having an aspiration aperture for communicating with a vacuum source; an ultrasonic transducer for generating ultrasonic vibrations; means for transmitting the ultrasonic vibrations to the aspiration tip; and control means for varying the vacuum pressure at the aspiration aperture.

Viewed from a third aspect this invention provides a bleed tube for attachment to an ultrasonic surgical aspirator handpiece, the bleed tube comprising: a first aperture for communicating with a vacuum source; a second aperture for connecting to the handpiece for communicating with an aspiration aperture in the handpiece; and a branching tube which, in use, overlies a gripping portion of the handpiece, the branching tube comprising a bleed aperture which, in use, can be partly or fully closed by covering with a finger or can be opened to bleed air to the vacuum source.

Viewed from a fourth aspect this invention provides an ultrasonic surgical aspirator comprising: a vacuum source; a handpiece comprising an aspiration tip with an aspiration aperture for communicating with the vacuum source; an ultrasonic transducer for generating ultrasonic vibrations; and means for transmitting the ultrasonic vibrations to the aspiration tip; one or more valves for selectively connecting the handpiece to the vacuum source or to an atmospheric air bleed; and a foot-switch for controlling the one or more valves and the ultrasonic transducer, whereby depression of the foot-switch controls the one or more valves to connect the handpiece to the vacuum source to provide a vacuum at the aspiration aperture and controls the ultrasonic transducer to generate ultrasonic vibrations.

This aspect of the invention may preferably be embodied by altering the momentary release part of the aspiration system such that the release is no longer momentary but continuous. Therefore, upon removal of the ultrasonic power the aspiration is also terminated.

Viewed from a fifth aspect this invention provides a handpiece for use in an ultrasonic surgical aspirator, the handpiece comprising: an aspiration tip having an aspiration aperture for communicating with a vacuum source; an ultrasonic transducer for generating ultrasonic vibrations; and means for transmitting the ultrasonic vibrations to the aspiration tip, the transmitting means being housed in an elongate prism-shaped shroud.

Embodiments of the invention will now be described, by way of example only, with reference to the accompanying drawings throughout which like parts are referred to by like references, and in which:

Figure 1 is a schematic diagram of a prior art ultrasonic surgical aspirator;

Figures 2a and 2b show a cross sectional plan view of a percutaneous ultrasonic handpiece according to a first embodiment of the invention;

Figure 3 shows a part sectional view of a laparoscopic surgical operation employing a percutaneous ultrasonic handpiece as shown in Figures 2a and 2b;

Figure 4 shows an enlarged part sectional end elevation of the handpiece of Figures 2a and 2b;

Figure 5 is an enlarged cross sectional side view of the rear of the handpiece of Figures 2a and 2b;

Figure 6 shows a part sectional side view of an ultrasonic handpiece according to a second embodiment of the invention;

Figure 7 shows a part sectional side view of an ultrasonic handpiece according to a third embodiment of the invention;

Figures 8, 9, 10 and 11 are plan views of the orifices shown in Figures 6 and 7; and

Figure 12 is a schematic diagram of an ultrasonic surgical aspirator according to a fourth embodiment of the invention;

Figure 13 shows the use of a laparoscopic ultrasonic handpiece for electrocautery; and

Figure 14 shows the use of a laparoscopic ultrasonic surgical handpiece in pulsed irrigation.

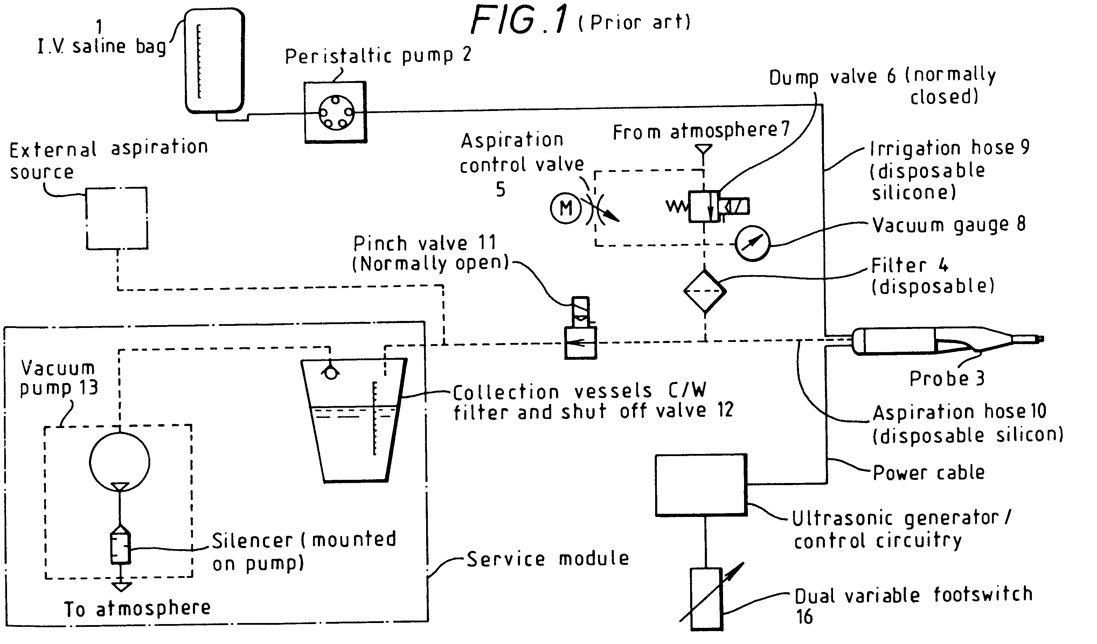

Referring now to Figure 1, a prior art ultrasonic surgical aspirator is shown in schematic form. The aspirator comprises an ultrasonic handpiece (or probe) 3 supplied with isotonic irrigation fluid from an intravenous (I.V.) bag via an irrigation hose 9. The irrigation fluid is drawn from the I.V. bag 1 by a peristaltic pump 2 connected to the irrigation hose 9.

Emulsified tissue, blood and irrigant are drawn from the tip of the handpiece 3 and into an aspiration hose 10 by means of a vacuum generated by a vacuum pump 13 or other external vacuum source. The rate at which emulsified tissue, blood and irrigant are drawn from the handpiece is controlled by an aspiration control valve 5 which controls a bleed of atmospheric air via an atmospheric air vent 7 into the aspiration hose 10. In parallel with the aspiration control valve 5 is a dump valve 6 which is normally closed, and which is synchronised with in operation with a pinch valve 11 which is normally open. The dump valve 6 and the pinch valve 11 are activated (made to change from their normal state of being open or closed) by one of two control signals from a foot-switch 16. The first of these signals, an irrigate only" signal, is supplied in response to the depression of an "irrigate only" pedal of the foot-switch, such that when a flush of irrigant at a high flow rate or priming of the irrigation hose is required, the aspiration is terminated. This is achieved by the dump valve 6 opening to bypass the aspiration control valve 5 to relieve the vacuum in the aspiration hose 10 up to the pinch valve 11 (which is simultaneously closed in response to the "irrigate only" signal). This condition is held so long as the "irrigate only" pedal is depressed. The second of the signals, a "momentary release" signal, is given at the point of release of a main ultrasonic power pedal of the foot-switch 16 and causes aspiration to be terminated momentarily by opening the dump valve 6 and simultaneously closing the pinch valve 11. This control is operated by the surgeon in anticipation of his wishing to remove the handpiece 3 from contact with the tissue. The period of release is controlled electronically to be nominally one second, the aspirator then reverting to the level of aspiration preset using the aspiration control valve 5.

The aspiration control valve 5, the dump valve 6, the atmospheric air vent 7 and a vacuum gauge 8 are housed in a control console and are protected from contamination by emulsified tissue or other debris by a filter 4 fitted into the aspiration hose 10. A service module is associated with the control console and comprises a collection vessel 12 (used to collect aspirated debris) and the vacuum pump 13. The vacuum pump 13 has a silencer to reduce its operating noise.

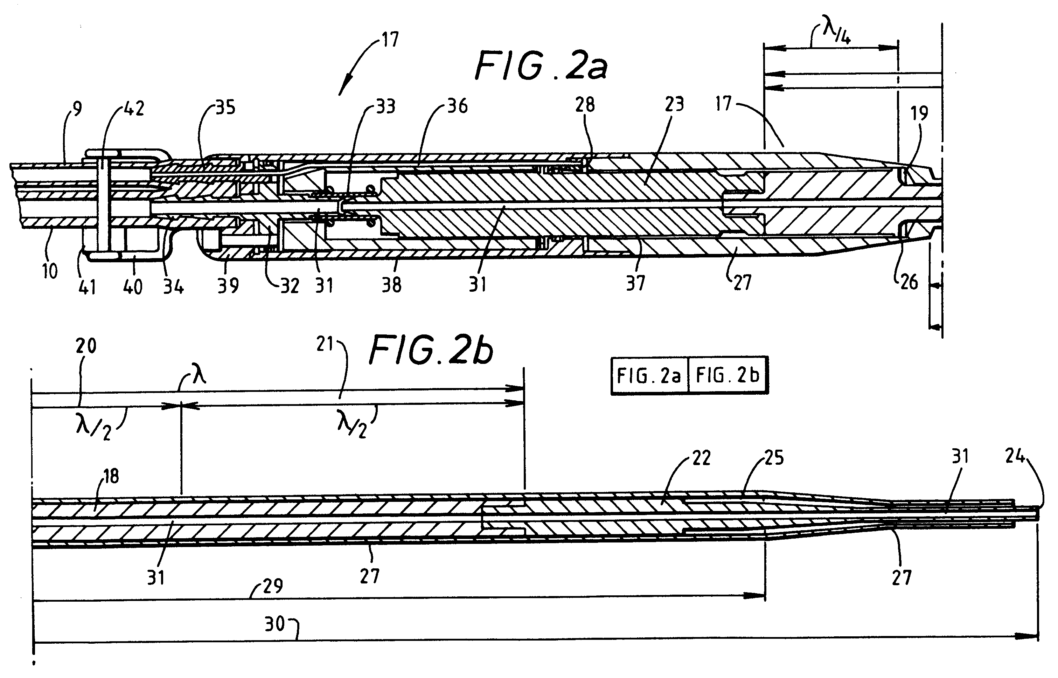

Figures 2a and 2b show a cross sectional plan view of a percutaneous (for use through the skin) ultrasonic handpiece 17 according to a first embodiment of the invention. The handpiece 17 is for use in conjunction with a laparoscope (a device for viewing the interior of the abdomen) for laparoscopic surgery.

The handpiece 17 comprises a handle section 38 housing an ultrasonic transducer 23 for generating ultrasonic vibrations. The handle is closed by an end cap 32. The ultrasonic vibrations are transmitted to a tip section 22 by a step amplifier 18.

To make the handpiece 17 suitable for use in laparoscopic surgery its length has been extended with respect to conventional handpieces. In order to provide the necessary reach within the pneumoperitoneum a step amplifier 18 is used which is one wavelength in length, where the wavelength is determined by the formula:-

$$\lambda = \frac{C}{F}$$

*where*
$\lambda = $ *wavelength*
$C = $ *speed of sound in the chosen material*
$F = $ *the chosen resonant frequency*

The step amplifier 18 can be considered as two half-wavelength sections 20, 21. The half-wavelength section 20 is attached to the transducer 23 and has an amplitude amplifying change at a position 19 (at or near the quarter-wavelength position). The second half-wavelength section 21 is a non-amplifying parallel extension of the half-wavelength section 20 and is manufactured in unitary form with the half-wavelength section 20.

A conventional three stage part exponential amplifying tip section 22 is screwed onto the end face of the step amplifier 18 remote from the transducer 23, and provides a working end 24. Raised shoulders (not shown) can be provided at a position 25 on the tip 22. In conjunction with similar raised shoulders at a position 26 on the step amplifier 18, these support a protective shroud 27 concentric with the vibrating parts. The raised shoulders are located at or near nodal points i.e. points of least vibration of the respective parts.

The protective shroud 27 is provided for the following reasons:

    a) to protect the patient from the ultrasonic vibrating parts 23, 18 and 22 (and vice versa);

    b) to provide an annular space 37 around the vibrating parts to deliver irrigant to the working end 24; and

    c) to provide a parallel (prism-shaped) surface along a length 29, of nominal diameter 10mm, to allow sealing against the inside surface of a standard introducer cannula.

By a suitable choice of material for the transducer 23, the step amplifier 18, and the tip 22, and a suitable choice of resonant frequency of operation, the length 30 from the position 26 of the raised shoulder to the working end 24 may be made suitable for most percutaneous surgical applications. For example, if titanium is used for the transducer 23, the step amplifier 18 and the tip 22, with a frequency of operation of 24 kHz, the length 30 would be nominally 265 mm.

The tip 22, the step amplifier 18, the transducer 23 and the end cap 32 are all provided with a co-joining axial aspiration passage 31. A flexible link 33 provides a means of linking the aspiration passage 31 across the interface between the vibrating rear end of the transducer 23 and the end cap 32. The aspiration passage 31 provides a path for emulsified tissue, blood and irrigant aspirated from an operating site adjacent to the distal working end 24 of the tip 22. The aspiration hose 10 (which is manufactured from a compliant material such as silicone) is push-fitted to an aspiration barbed connector 34 on the endcap 32. The irrigation hose 9 is also manufactured from a compliant material such as silicone, and is co-extruded with the aspiration hose 10 to form a "figure-of-eight" configuration. The aspiration and irrigation hoses are thus held adjacent to one another between the handpiece 17 and the control console.

The irrigation hose 9 is push fitted, simultaneously with the aspiration hose 10, onto an irrigation barbed connector 35. In use, irrigant flows through the irrigant hose 9, into the irrigant barbed connector 35, through a tube 36 and the port 28, into the annular space 37 and out into the operating site at the working end 24.

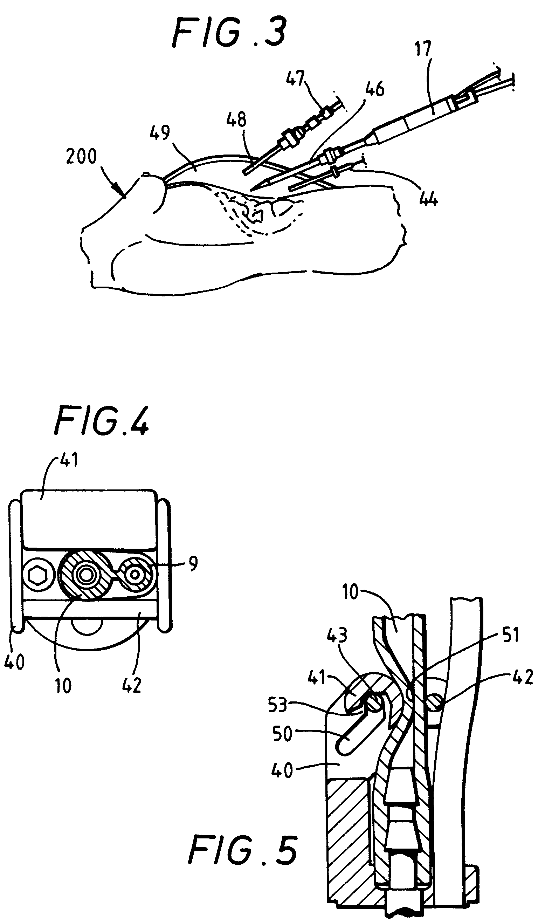

An endcap cover 39 covers the end of the handpiece and provides a convenient component on which to mount an aspiration clamp fork 40. As described below with reference to Figures 4 and 5, the aspiration clamp fork 40 provides the surgeon with a control of the aspiration level. (An alternative is to manufacture the aspiration clamp fork 40 and the endcap cover 39 as a single unit; this alternative is described with reference to Figure 5 below).

Figure 3 shows a part sectional view of a laparoscopic surgical operation employing a percutaneous ultrasonic handpiece 17 as shown in Figures 2a and 2b. The outer wall of the abdomen of a patient 200 is inflated through an insufflator cannula 44 to provide a working space 49, (the pneumoperitoneum). A cannula 46 is inserted through a small incision and allows the fitting and sealing through of the percutaneous ultrasonic handpiece 17. A cannula 48 allows the fitting and sealing through of a laparoscope 47 for illuminating and viewing the operating site. Although the percutaneous ultrasonic handpiece 17 may be referred to as a "laparoscopic" handpiece it is not strictly laparoscopic since a separate device (the laparoscope 47) is used for viewing and illuminating scope the operating site. Thus, although a handpiece with a concentric or adjacent parallel viewing and illuminating apparatus could be constructed, the combination of the handpiece 17 and the laparoscope 47 shown in Figure 3 can provide better vision and perspective of the operating site.

Figures 4 and 5 respectively show an enlarged part sectional end elevation and an enlarged cross sectional side view of the rear of the handpiece 17 described with reference to Figures 2a and 2b. Control of the aspiration level applied at any time is given to the surgeon by means of the aspiration clamp fork 40.

A roller 41 is attached to the aspiration clamp fork 40 by a pin 43. The pin 43 is free to slide or roll in two parallel slots 50 which are machined in each leg of the aspiration clamp fork 40. The roller 41 is moved in the slots 50 by thumb action to crimp the aspiration hose 10 at a point 51 between the roller 41 and a pin 42. The pitch between the roller 41 and the pin 42 in the closed position selected to ensure substantially complete pinching of the aspiration hose 10 while allowing for manufacturing tolerances in the hose size. The length and angle of the slots 50 is selected to ensure closure can be activated by the normal extension of the thumb. The slots 50 are also provided with shoulders 53 so that the roller 41 can be latched in the closed position. The roller 41 has a knurled outside surface to provide a high friction surface. Straight large pitch knurling is used to facilitate cleaning.

Figure 5 shows the roller 41 in the closed position and Figure 4 shows the roller 41 in the open position. In Figure 5, the aspiration clamp fork 40 is shown as an integral part of the endcap cover 39.

Using his thumb to move the roller 40 into a position in which it crimps the aspiration hose 10, the surgeon is able to terminate aspiration at the working end 24 of the handpiece tip 22, thus ceasing to reduce the pneumoperitoneum and allowing the insufflator to maintain the required inflation of the abdomen. This means that the surgeon does not to have to withdraw the ultrasonic handpiece from a patient each time he temporarily ceases to perform ultrasonic surgery.

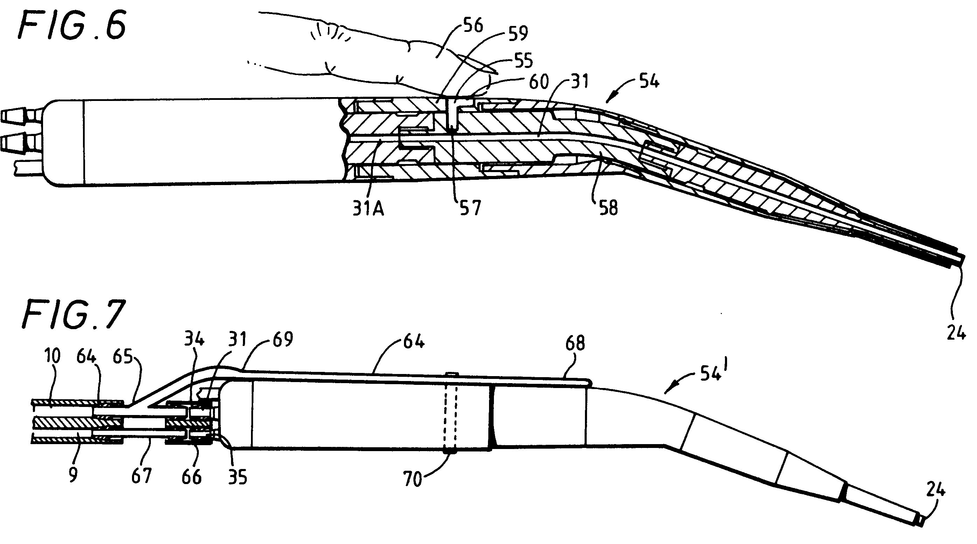

Figure 6 shows a part sectional side view of an ultrasonic handpiece 54 according to a second embodiment of the invention. In the handpiece 54 the aspiration passage 31 is provided with a bleed port 57 connected to a bleed valve 55 which can be covered or uncovered by the surgeon's finger 56. The maximum working aspiration level is preset at the control console by aspiration control valve 5 (Figure 1). With the finger 56 occluding the bleed valve 55 aspiration is performed in the normal way, in that debris withdrawn from the working end 24 of the tip 22 passes along the aspiration passage 31 and eventually into the collection vessel 12 (Figure 1).

However, by moving the finger 56 to partly or wholly open the bleed valve 55, a bleed of atmospheric air is allowed into the aspiration passage. This reduces the aspiration vacuum at the working end 24 of the tip 22. Control of the aspiration level at the working end 24 is thus given to the surgeon. Giving control of the aspiration level to the surgeon in this manner is also advantageous in e.g. neurosurgery (surgery of the nervous system, and in particular the brain) where highly delicate tissue structures can be very sensitive to the aspiration level.

If the bleed port 57 is suitably large with respect to the cross sectional area of the aspiration passage at a position 31a, then with the finger completely removed (and the bleed valve 55 completely uncovered) aspiration at the working end 24 can be reduced to substantially zero. The bleed port 57 and the bleed valve 55 may be provided at a convenient point and orientation on the handpiece. The bleed port 57 is preferably made from a compliant material such as silicone so as not to transmit vibrations from the vibrating transducer arrangement to the external surface. Such a compliant material also provides a leak-tight fit between (a) the step amplifier 58 and the port 57 and (b) the shroud 59 and the port 57.

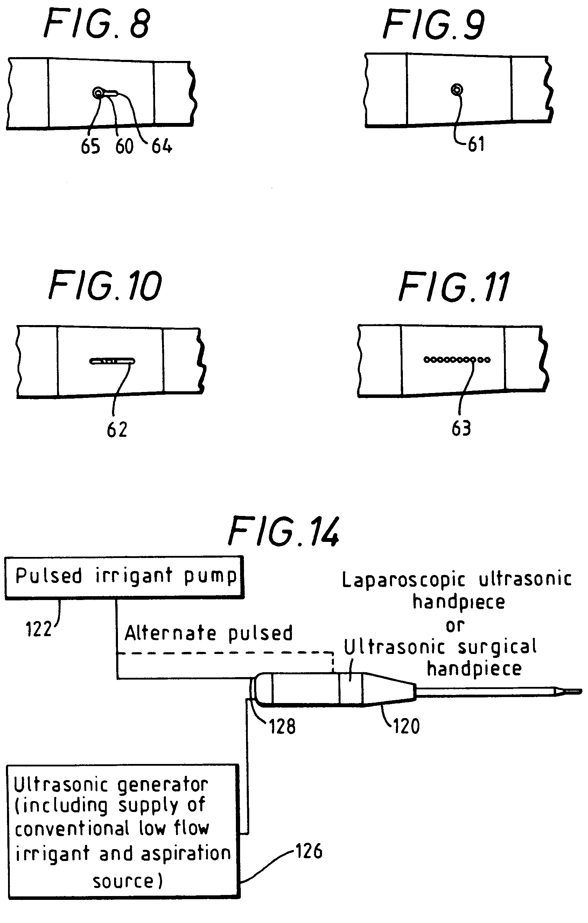

The bleed valve 55 comprises a shaped orifice 60 which can be partly or wholly covered by the surgeon's finger 56. The shape of the orifice 60 may be one of a number of different configurations to provide various degrees of control over the aspiration level at the working end 24. A number of examples of shaped orifices are shown in Figures 8, 9, 10 and 11.

Figure 7 shows a part sectional side view of an ultrasonic handpiece according to a third embodiment of the invention. An external tube 64 is "piggy backed" onto a handpiece 54' and is ported into the aspiration passage 31 between the aspiration barbed connector 34 and the aspiration hose 10 by a T-piece fitting 65. The tube 64 is made from suitably sized round stainless steel stock hypodermic tube or plastic tube. It is reduced at a point 69 from a round cross section to a elliptical or oval shape to reduce the protrusion it makes above the outside profile of the handpiece 54'. The tube 64 is provided with an orifice 68 which can take the form, for example, of any of the orifices shown in Figures 8, 9, 10 or 11. Two link pieces 66 and 67 are provided to join the irrigation barbed connector 35 to the irrigation hose 9. The link piece 66 has a "figure-of-eight" formation and also joins the aspiration barbed connector to the T-piece fitting 65 of the tube 64, which in turn is connected to the aspiration hose 10. A clip or "0" ring 70 (or another suitable retaining device) is provided to hold the tube 64 onto the back of the handpiece 54'.

Figure 8 shows in plan view a first shaped orifice (in fact the orifice 60 which is illustrated in side elevation in Figure 6), which provides a degree of variable control over aspiration level at the working end 24. The orifice 60 comprises a slotted front section 64, which widens towards the rear of the orifice 60 to form a broadened rear section 65. Assuming that the orifice 60 is initially covered by the surgeon's finger 56, the finger can be drawn back in stages to expose first the slotted front section (thus providing a limited degree of attenuation of the aspiration level) and then the broadened rear section 65. The slotted front section 64 and the broadened rear section 65 provide in total a sufficiently large orifice area to provide a significant air bleed into the aspiration passage 31.

Figure 9 shows a circular orifice 61 which provides an on/off control, by means of the surgeon's finger either fully covering or fully uncovering the circular orifice 61.

Figure 10 shows another alternative, in which a long slotted orifice 62 requires a large movement of the finger 56 for a given change in aspiration level. This arrangement provides a fine control over the aspiration level at the working end 24. The total area of the orifice 62, when fully uncovered, is sufficient that the aspiration level at the working end 24 can be reduced to substantially zero.

Figure 11 shows a further alternative orifice shape 63, in which a series of holes is provided to work in a similar manner to the slotted orifice 62. The spacing of the holes can be selected to require still further finger travel than that required for the slotted orifice 62, which means that the aspiration control can be made even less sensitive to finger movement. This arrangement potentially allows very fine control over the aspiration level at the working end 24. The lack of sensitivity of the control to small finger movements can be useful in e.g. neurosurgery when the surgeon's full attention is required by the surgical procedures themselves.

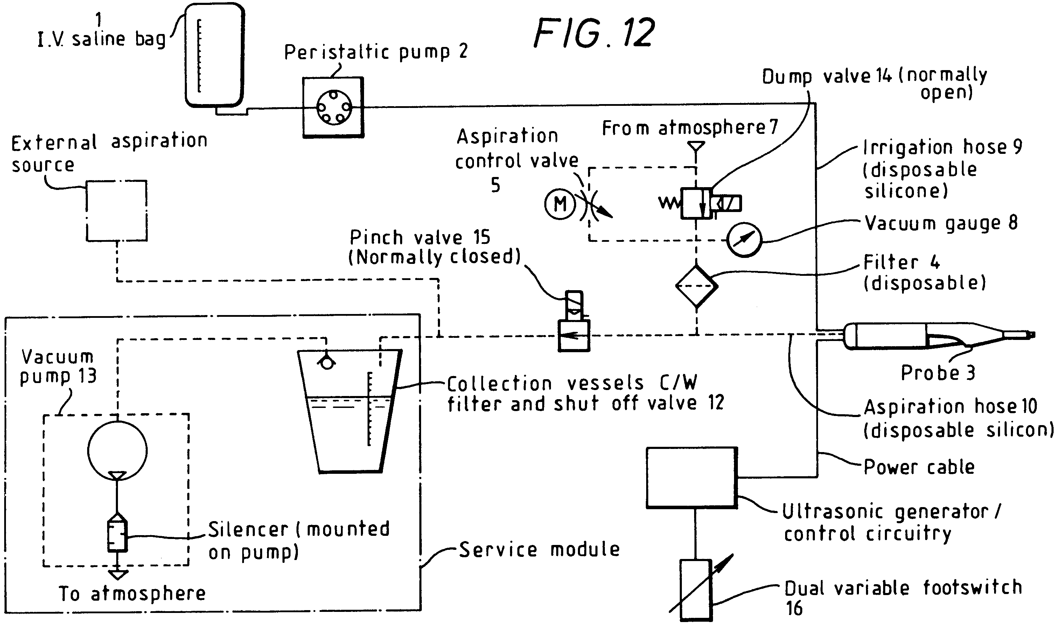

Figure 12 is a schematic diagram of an ultrasonic surgical aspirator according to a fourth embodiment of the invention. In this arrangement the polarity of a dump valve 14 and a pinch valve 15 are reversed so that the dump valve 14 is normally open and the pinch valve 15 is normally closed. This measure reduces the aspiration vacuum supplied to the handpiece 3 (i.e. downstream of the pinch valve 15) to near zero, so as not to deflate the working space during a laparoscopic surgical operation.

The dump valve 14 and the pinch valve 15 are now operated solely by an "aspirate" signal from the main ultrasonic power pedal of the foot-switch 16'. The "aspirate" signal is given by the pedal at the moment depression of the pedal is started, and causes the dump valve 14 and the pinch valve 15 to change state (i.e. from open to closed or vice-versa). This restores the aspiration vacuum supplied to the handpiece 3 to the level preset by the aspiration control valve 5. It is no longer necessary to employ an "irrigate only" signal from the "irrigate only" pedal, because the dump valve 14 and the pinch valve 15 are normally in a suitable state for "irrigate only" operation.

The vacuum pump 13 is provided with a small bleed hole (nominally 0.7mm diameter) on its inlet to ensure the pump does not pull continuously against an occluded aspiration hose. This serves to maintain the life of the pump and reduce the operational noise.

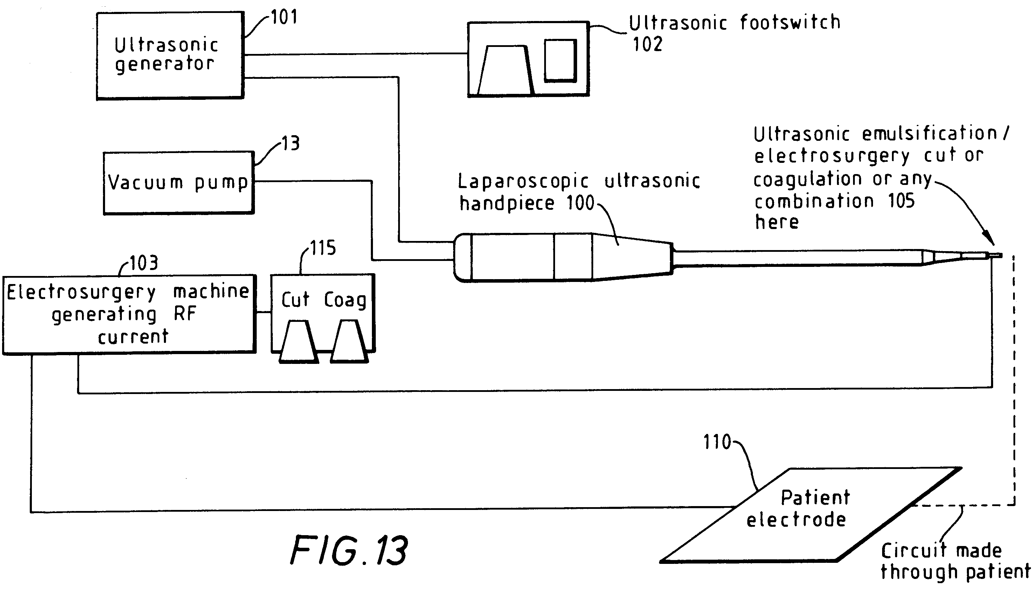

Figure 13 shows the use of a laparoscopic ultrasonic handpiece 100 (controlled by an ultrasonic generator 101 and an ultrasonic foot-switch 102) with the addition of an electrocautery facility. Electrocautery is used to provide haemostasis (blood-clotting) of tissue and/or blood vessels by applying a radio frequency signal of high voltage, generated by a standard electrosurgery machine 103, to the ultrasonic handpiece tip 105. The flow of current and therefore the cauterising effect is controlled:

a) by the electrical resistance of an electrical circuit through the patient via the handpiece tip 105 and a large patient plate electrode 110 (the latter is placed under the patients body and in contact with the skin); and

b) by a foot-switch 115 operable to switch the electrical circuits on or off.

The standard construction of a piezoelectric transducer is such that the metallic parts in electrical connection with the tip are isolated to restrict the patient leakage current flowing when using the handpiece as an ultrasonic tool. This isolation also provides protection to the ultrasonic circuit from the electrocautery voltage, allowing the electrocautery voltage to be applied direct to the tip, either on its own or in combination with ultrasonic vibration to affect various forms of tissue cutting, coagulation, and selective removal.

Figure 14 shows the use of a laparoscopic ultrasonic surgical handpiece 120, with the addition of a pulsed irrigation facility. This is the application of a pulsed flow of relatively high flow isotonic irrigant to an operating site. The pulsed irrigant is used to dislodge loose tissue and/or coagulated blood clots that may be obscuring the surgeon's view of certain parts of the operative site.

The ultrasonic handpiece 120 combines ultrasonic operation with pulsed irrigant delivery in a single unit, and is connected to a pulsed irrigant pump 122, and an ultrasonic generator 126 (including a vacuum pump for aspiration). The ultrasonic generator 126 also supplies conventional (non-pulsed) irrigant.

In laparoscopic or endoscopic surgery it is preferable to use the minimum number of cannula ports into the patient and also to limit the number of times instruments have to be removed and inserted or swapped within these cannulae. The handpiece 120 combines pulsed irrigation and ultrasonic operation into a common instrument incorporating an additional irrigant port 128 at the rear of the handpiece to allow the connection of the pulsed irrigant pump 122.

**Claims**

1. An ultrasonic surgical aspirator comprising a vacuum source (13) and a handpiece (17), the handpiece (17) comprising:

   an aspiration tip (22) with an aspiration aperture for communicating with the vacuum source (13);

   an ultrasonic transducer (23) for generating ultrasonic vibrations;

   means (18) for transmitting the ultrasonic vibrations to the aspiration tip (22); and

   control means (40, 41) for varying the vacuum pressure at the aspiration aperture.

2. An aspirator according to claim 1, comprising:

   an irrigant pump (2) for supplying irrigant fluid to the handpiece;

   and in which the handpiece comprises an irrigant outlet aperture for communicating with the irrigant pump.

3. An aspirator according to claim 2, comprising means (122) for supplying a pulsed flow of irrigant fluid to the irrigant outlet aperture.

4. An aspirator according to any one of the preceding claims, in which the control means (40, 41) comprises means for restricting a path of fluid flow between the aspiration aperture and the vacuum source, so as to vary the vacuum pressure at the aspiration aperture.

5. An aspirator according to claim 4, in which the handpiece (17) is connected to the vacuum source (13) by a flexible aspiration hose (10), and in which the control means (40, 41) comprises means for crimping the aspiration hose (10) to restrict a path of fluid flow between the aspiration aperture and the vacuum source (13).

6. An aspirator according to claim 5, in which the crimping means comprises a roller (41) movable with respect to the aspiration hose (10) between a crimping position and a non-crimping position.

7. An aspirator according to claim 6, in which the handpiece (17) comprises means (53) for latching the roller in the crimping position.

8. An aspirator according to any one of claims 1 to 3, in which the control means comprises means (57) for bleeding air to the vacuum source.

9. An aspirator according to claim 8, in which the handpiece (17) comprises a bleed aperture (57) which, in use, can be partly or fully closed by covering with a finger or can be opened to bleed air to the vacuum source (13).

10. An aspirator according to claim 9, in which the handpiece (17) comprises a plurality of spaced bleed apertures (63).

11. An aspirator according to claim 9, in which the bleed aperture (62) is elongate in shape.

12. An aspirator according to claim 8, in which the control means comprises a bleed tube (64) attached to the handpiece (17), the bleed tube (64) comprising:

    a first aperture for communicating with the vacuum source (13);

    a second aperture connected to the handpiece (17) for communicating with the aspiration aperture in the handpiece; and

    a branching tube which, in use, overlies a gripping portion of the handpiece, the branching tube comprising a bleed aperture (68) which, in use, can be partly or fully closed by covering with a finger or can be opened to bleed air to the vacuum source (13).

13. An aspirator according to any one of the preceding claims, in which the transmitting means is housed in an elongate prism-shaped shroud (27).

14. An aspirator according to claim 13, in which the handpiece (17) is connectable to an electrocautery signal generator (103), the aspiration tip forming an electrocautery electrode when the handpiece is connected to the electrocautery signal generator.

15. A handpiece (17) for use in an ultrasonic surgical aspirator, the handpiece comprising:

    an aspiration tip (22) having an aspiration aperture for communicating with a vacuum source (13);

    an ultrasonic transducer (23) for generating ultrasonic vibrations;

    means (18) for transmitting the ultrasonic vibrations to the aspiration tip; and

    control means (40, 41) for varying the vacuum pressure at the aspiration aperture.

16. A bleed tube (64) for attachment to an ultrasonic surgical aspirator handpiece (17), the bleed tube comprising:

    a first aperture for communicating with a vacuum source (13);

    a second aperture for connecting to the handpiece (17) for communicating with an aspiration aperture in the handpiece; and

    a branching tube which, in use, overlies a gripping portion of the handpiece, the branching tube comprising a bleed aperture (68) which, in use, can be partly or fully closed by covering with a finger or can be opened to bleed air to the vacuum source (13).

17. An ultrasonic surgical aspirator comprising:

a vacuum source (13);

a handpiece (17) comprising an aspiration tip (22) with an aspiration aperture for communicating with the vacuum source (13); an ultrasonic transducer (23) for generating ultrasonic vibrations; and means (18) for transmitting the ultrasonic vibrations to the aspiration tip;

one or more valves (5, 14, 15) for selectively connecting the handpiece (17) to the vacuum source (13) or to an atmospheric air bleed (7); and

a foot-switch (16) for controlling the one or more valves (5, 14, 15) and the ultrasonic transducer (23), whereby depression of the foot-switch (16) controls the one or more valves (5, 14, 15) to connect the handpiece (17) to the vacuum source (13) to provide a vacuum at the aspiration aperture and controls the ultrasonic transducer (23) to generate ultrasonic vibrations.

18. A handpiece (17) for use in an ultrasonic surgical aspirator, the handpiece comprising:

an aspiration tip (22) having an aspiration aperture for communicating with a vacuum source (13);

an ultrasonic transducer (23) for generating ultrasonic vibrations; and

means (18) for transmitting the ultrasonic vibrations to the aspiration tip, the transmitting means being housed in an elongate prism-shaped shroud (27).

19. A handpiece according to claim 18, the handpiece (17) being connectable to an electrocautery signal generator (103), the aspiration tip forming an electrocautery electrode when the handpiece (17) is connected to the electrocautery signal generator.

**FIG.1** (Prior art)

1 I.V. saline bag

Peristaltic pump 2

External aspiration source

Aspiration control valve 5

From atmosphere 7

Dump valve 6 (normally closed)

Irrigation hose 9 (disposable silicone)

Vacuum gauge 8

Filter 4 (disposable)

Pinch valve 11 (Normally open)

Vacuum pump 13

Collection vessels C/W filter and shut off valve 12

Probe 3

Aspiration hose 10 (disposable silicon)

Power cable

Silencer (mounted on pump)

Service module

Ultrasonic generator / control circuitry

To atmosphere

Dual variable footswitch 16

EP 0 578 376 A1

FIG.2a

FIG.2b

| FIG.2a | FIG.2b |

EP 0 578 376 A1

## FIG. 3

## FIG. 4

## FIG. 5

FIG.6

FIG.7

EP 0 578 376 A1

# FIG. 8

65 60 64

# FIG.9

61

# FIG.10

62

# FIG.11

63

# FIG.14

Pulsed irrigant pump

122

Alternate pulsed

128 120

Laparoscopic ultrasonic handpiece
or
Ultrasonic surgical handpiece

Ultrasonic generator (including supply of conventional low flow irrigant and aspiration source)

126

FIG.12

I.V. saline bag 1

Peristaltic pump 2

External aspiration source

Dump valve 14 (normally open)

Irrigation hose 9 (disposable silicone)

From atmosphere 7

Vacuum gauge 8

Filter 4 (disposable)

Probe 3

Aspiration hose 10 (disposable silicon)

Power cable

Ultrasonic generator / control circuitry

Dual variable footswitch 16

Aspiration control valve 5

Pinch valve 15 (Normally closed)

Collection vessels C/W filter and shut off valve 12

Service module

Silencer (mounted on pump)

Vacuum pump 13

To atmosphere

Ultrasonic footswitch 102

Ultrasonic emulsification / electrosurgery cut or coagulation or any combination 105 here

Laparoscopic ultrasonic handpiece 100

Circuit made through patient

Patient electrode

110

Ultrasonic generator

101

Vacuum pump

13

115

Cut Coag

Electrosurgery machine generating RF current

103

17

FIG.13

EP 0 578 376 A1

**European Patent Office**

## EUROPEAN SEARCH REPORT

Application Number

| | DOCUMENTS CONSIDERED TO BE RELEVANT | | EP 93304462.0 |
|---|---|---|---|

| Category | Citation of document with indication, where appropriate, of relevant passages | Relevant to claim | CLASSIFICATION OF THE APPLICATION (Int. Cl.5) |
|---|---|---|---|
| X | EP - A - 0 384 672 (H. TAKASE) * Totality; especially column 4, line 29 - column 5, line 8 * | 1,8,9, 13,15 | A 61 B 17/32 A 61 M 1/00 |
| Y | | 2,4-7, 10,11, 14 | |
| A | | 12 | |
| X | GB - A - 2 176 110 (NIPPON INFR. IND.) * Totality; especially fig. 1; page 2, lines 30-38 * | 1,8,9, 15 | |
| Y | | 2,4-7, 10,11, 14 | |
| A | | 12,16 | |
| X | US - A - 4 136 700 (A. BROADWIN ET AL.) * Totality; especially column 5, line 25 - column 6, line 4; fig. 1 * | 17,18 | TECHNICAL FIELDS SEARCHED (Int. Cl.5) |
| Y | | 2 | A 61 B |
| A | | 1 | A 61 M |
| X | US - A - 4 750 902 (D.W. WUCHINICH ET AL.) * Fig. 12B,13; column 13, lines 55-62; column 16, line 65 - column 17, line 6; column 20, line 37 - column 21, line 7 * | 17-19 | |
| A | | 1 | |
| Y | | 14 | |
| Y | EP - A - 0 150 666 (PFISTER-LEHMANN) * Fig. 7,8; claims 1,11,12 * | 4-7 | |

The present search report has been drawn up for all claims

| Place of search | Date of completion of the search | Examiner |
|---|---|---|
| VIENNA | 08-10-1993 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## EUROPEAN SEARCH REPORT

European Patent Office

Application Number

EP 93304462.0

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP - A - 0 463 798 (VANCE PROD.) * Fig. 1-3; column 3, lines 37-48; claim 8 * | 10 | |
| Y | US - A - 4 878 900 (T.M. SUNDT) * Fig. 1,2; abstract; column 2, lines 25-31 * | 11 | |
| Y | US - A - 4 931 047 (A. BROADWIN ET AL.) * Totality; especially abstract; column 3, lines 11-20; claim 1 * | 14 | |
| Y | US - A - 4 886 060 (H. WIKSELL) * Totality; especially fig. 1; column 2, lines 25-41 * | 14 | |
| A | US - A - 5 022 414 (G.H. MULLER) * Claims 1,8,9,11,13,19,23 * | 3 | TECHNICAL FIELDS SEARCHED (Int. Cl.5) |
| A | EP - A - 0 174 084 (SURG. DYN.) * Fig. 1-3; page 8, lines 7-20 * | 3 | |

The present search report has been drawn up for all claims

| Place of search | Date of completion of the search | Examiner |
|---|---|---|
| VIENNA | 08-10-1993 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)